# EUROPÄISCHE PATENTSCHRIFT

(11) **EP 3 553 787 B1**
(45) Veröffentlichungstag und Bekanntmachung des Hinweises auf die Patenterteilung: **31.07.2024**
(21) Anmeldenummer: 19168892.8
(22) Anmeldetag: 12.04.2019
(51) Int. Cl.: G16H 40/63, G16H 40/67, G16H 20/40, G16H 10/60

(54) **SYSTEM ZUR DATENÜBERTRAGUNG**
DATA TRANSFER SYSTEM
SYSTÈME DE TRANSMISSION DE DONNÉES

(30) Priorität: 12.04.2018 DE 102018002997
(43) Veröffentlichungstag der Anmeldung: 16.10.2019
(73) Patentinhaber: Löwenstein Medical Technology S.A., 2557 Luxembourg (LU)
(72) Erfinder: Schwaibold, Matthias, 76228 Karlsruhe (DE)
(74) Vertreter: Marx, Thomas

(56) Entgegenhaltungen:
- WO-A1-2007/030457
- DE-T2- 69 634 169
- US-A- 5 921 938
- US-A1- 2009 081 951
- US-A1- 2009 163 793
- US-A1- 2012 310 669
- US-A1- 2014 000 609

## Beschreibung

Die vorliegende Erfindung betrifft ein System zur Datenübertragung zwischen einem Server und einem Beatmungsgerät. Im Stand der Technik ist die Therapie mit Beatmungsgeräten bekannt, wobei zunehmend Beatmungsgeräte digitale Schnittstellen zum Datenaustausch aufweisen. Dabei umfassen die Beatmungsgeräte in der Regel mindestens eine Schnittstelle zur Ermöglichung einer Datenübertragung. Zum Datenaustausch sind die Beatmungsgeräte dabei oftmals mit einer Schnittstelle zum Datenaustausch mit einem Netzwerk ausgerüstet. Im Stand der Technik ist ganz, dass eine solche Schnittstelle kabelgebunden, beispielsweise als RJ45 Datenschnittstelle ausgeführt, oder kabellos, beispielsweise als WLAN, Mobilfunk-, IoT, M2M oder Bluetooth Schnittstelle ausgeführt sein kann.

Über eine solche Datenschnittstelle können eine Vielzahl von Daten ausgetauscht werden. Beispielsweise kann die Software des Beatmungsgerätes durch ein Update durch Zugriff auf entfernte Daten aktualisiert werden, wie in der DE 10 2015 008 946 A1 beschrieben.

Weiterhin können Behandlungsdaten, wie Einstellungen, Messwerte therapeutischer oder physiologischer Parameter oder auch Patientendaten an ein entferntes Gerät, beispielsweise an einen Server zur Abspeicherung und Verfügbarmachung für Dritte gesendet werden, wie in der EP 2 392 253 A1 beschrieben.

In der DE 696 34 169 T2 wird ein Beatmungsgerät beschrieben, welches mit einer Datenerfassungseinrichtung ausgestattet ist, welche Daten vom Motor, Batterie, Durchflussmesser und Drucksensoren erhält mit einem Zeitstempel versieht. Die Daten der Datenerfassungseinrichtung werden von Zeit zu Zeit die gesammelten Daten in eine Datenbank hochgeladen, um eine Protokollierung sowie weitere Analyse zu ermöglichen.

Die US 2014/000609 A1 offenbart ebenfalls ein Beatmungsgerät, welches Möglichkeiten zur Datenspeicherung, welche gesammelte Daten mit einem Zeitstempel versieht, aufweist. Die gesammelten Daten können zwischen dem Beatmungsgerät und etwa einem Server in Echtzeit oder auch von Zeit zu Zeit übertragen werden.

Eine weitere Einrichtung zur Datenerfassung und Datenübertragung im medizintechnischen Bereich wird in der WO 2007/030457 A1 gezeigt. Die Daten werden von einer Messeinrichtung erfasst und mit einem Zeitstempel versehen. Beim Übertragen der Daten wird die Systemzeit der Messeinrichtung mit der Prozessierungs- bzw. Speichereinrichtung verglichen und der Zeitstempel der Daten gegebenenfalls angepasst.

Die Anpassung der Zeitstempel kann auch auf der Differenz zwischen dem medizinischen Instrument und einem externen Zeitgeber basieren, wie die US 5 921 938 A offenbart. Dabei wird die Systemzeit des medizinischen Instruments mit der Zeit des Zeitgebers verglichen und die Differenz der Zeiten auf die Zeitstempel der Daten angewendet und auch die Systemzeit des medizinischen Instruments wird gegebenenfalls mit der Zeit des Zeitgebers synchronisiert, damit die weiteren aufgenommenen Daten einen konsistenten Zeitstempel aufweisen.

Die aus dem Stand der Technik bekannten Lösungen weisen jedoch den Nachteil auf, dass bei Daten, insbesondere Patientendaten, die zu einer weiteren Auswertung benötigt werden, bei einem Systemfehler, beispielsweise einer falsch eingestellten Uhrzeit, die sich beispielsweise bei einer Umstellung von Sommer- auf Winterzeit ergeben kann, falsch zugeordnet werden. Beispielsweise können Daten als an einem anderen Tag erhoben angezeigt werden. Dies verursacht erhebliche Probleme bei der Zuordnung der Daten bei der Übertragung.

Insbesondere, wenn die erhobenen Daten nicht in Echtzeit übertragen werden, sondern zu einem späteren Zeitpunkt übertragen werden sollen, kann eine falsche Uhrzeit dazu führen, dass die übertragenen Daten nicht zuordenbar und damit unbrauchbar sind.

Daher ist es Aufgabe der vorliegenden Erfindung, ein System zur Datenübertragung bereitzustellen, dass sicherstellt, dass die erhobenen Daten mit korrekten Zeitstempel versehen und übertragen werden.

Diese Aufgabe wird durch ein System zur Datenübertragung gemäß dem erfindungsgemäßen Anspruch 1 gelöst.

Gegenstand der vorliegenden Erfindung ist ein System zur Datenübertragung zwischen einem Server und einem Beatmungsgerät, wobei das Beatmungsgerät aufweist; eine Quelle für Atemgas und zumindest eine Schnittstelle zur Ermöglichung einer Datenübertragung, eine Vorrichtung zur Ermittlung von Daten, eine Steuereinheit, einen Speicher, eine Zeiteinheit zur Festlegung einer Systemzeit, wobei das Beatmungsgerät die Daten zumindest zeitweise in dem Speicher ablegt und den Daten die Systemzeit als Zeitstempel zuordnet, wobei die Systemzeit über die Schnittstelle an den Server versendet wird, wobei der Server eine Server-Zeiteinheit zur Bereitstellung einer Server-Zeit und eine Vergleichseinrichtung aufweist, wobei die Vergleichseinrichtung die Server-Zeit mit der Systemzeit vergleicht und bei einer definierten Übereinstimmung der Server-Zeit mit der Systemzeit die Daten von dem Beatmungsgerät überträgt und in einem Serverspeicher ablegt und wobei der Server eingerichtet ist, eine Abweichung zwischen der Systemzeit und der Server-Zeit zu bewerten und eine entsprechende Bewertung an das Beatmungsgerät zu übermitteln, wobei das Beatmungsgerät bei einer definierten Abweichung der Server-Zeit von der Systemzeit eine Übertragung von Daten von dem Beatmungsgerät sperrt, bis die Systemzeit derart neu festgelegt wird, dass eine definierte Übereinstimmung mit der Server-Zeit erreicht ist, wobei die neu festgelegte Systemzeit derart auf den Zeitstempel in dem Speicher des Beatmungsgerätes angewandt wird, dass den Daten ein neuer Zeitstempel zugeordnet wird, woraufhin die Daten mit dem neuen Zeitstempel an den Server übertragen werden.

Die Datenübertragung kann beispielsweise eine Übertragung von Tages- Statistikwerten, die nach Ablauf eines Tages Stück übertragen werden. Bei einer Störung der Übertragung, werden die Tages- Statistikwerte gespeichert und an einem anderen Tag zusammenhängend übertragen. Alternativ kann eine Datenübertragung wird eine Applikation durchgeführt werden. Eine weitere Alternative ist der Datenaustausch Diagnosegeräten.

Das Beatmungsgerät ist eingerichtet, die Daten mit einem Zeitstempel zu versehen, zu speichern, und zu einem späteren Zeitpunkt zu übertragen, wobei der Zeitstempel nicht oder nur begrenzt auf ein Toleranzfenster einstellbar eingerichtet ist. Dies bietet den Vorteil, dass feststellbar ist, zu welchem Datum und Zeitpunkt die Daten aufgezeichnet wurden bzw. die Therapie durchgeführt wurde. Zudem ist es möglich sicherzustellen, dass das Datum und die Uhrzeit in dem Beatmungsgerät hinreichend korrekt sind, ohne dass sie manuell einstellbar sind. Zu einem späteren Zeitpunkt bedeutet, dass das Beatmungsgerät eingerichtet ist, die Daten nicht in Echtzeit zu übertragen, sondern in einem Speicher zwischenzuspeichern und zu einem zu späteren Zeitpunkt zu übertragen. In der Regel wird der Zeitstempel von einem externen Zeitbereitstellungsdienst, beispielsweise eine Zeit-Server, vorgegeben. Typischerweise ist das Beatmungsgerät eingerichtet, mit dem Zeitbereitstellungsdienst mindestens einmalig zu kommunizieren. Der erzeugte Zeitstempel wird zu den jeweiligen Daten auf dem Beatmungsgerät gespeichert und bei einer Übertragung zusammen mit den Daten übermittelt.

In einer Weiterbildung ist das System eingerichtet, basierend auf dem Zeitstempel einen Abgleich einer Systemzeit des Beatmungsgerätes mit einer Server-Zeit des Servers durchzuführen, wenn die Datenübertragung gestartet wird. Eine Datenübertragung wird auch als Telemonitoring bezeichnet. Die Systemzeit wird mittels des Zeitstempels definiert. Die Server-Zeit wird durch den Server definiert. Bei einer beginnenden Datenübertragung/einem beginnenden Telemonitoring ist das System eingerichtet, den Zeitstempel bzw. die Systemzeit des Beatmungsgerätes dem Server mitzuteilen.

In einer weiteren Weiterbildung ist das Beatmungsgerät eingerichtet, eine Systemzeit von dem Server zu empfangen du die Systemzeit automatisch einzustellen. Dies bietet den Vorteil, dass die korrekte Zeit automatisch eingestellt wird.

In einer weiteren Ausgestaltung ist der Server eingerichtet, eine Abweichung zwischen der Systemzeit und der Server-Zeit zu bewerten und eine entsprechende Bewertung an das Beatmungsgerät zu übermitteln. Der Server ist eingerichtet, die von dem Beatmungsgerät gesendeten Daten, insbesondere den Zeitstempel bzw. die Systemzeit zu empfangen. Der Server ist eingerichtet, die Systemzeit mit der Server-Zeit zu vergleichen und eine Abweichung festzustellen. Optional kann der Server eingerichtet sein, ein Toleranzfenster vorzuhalten, in dem abweichende Zeiten (Systemzeit von Server-Zeit) toleriert werden und vom Server als korrekt angesehen werden. Beispielsweise kann ein solches Toleranzfenster zwischen 0,1 Sekunden bis 1 Minute betragen.

In einer Weiterbildung ist das Beatmungsgerät eingerichtet, eine Anmeldung des Beatmungsgeräts an dem Server basierend auf der Bewertung der Systemzeit durch den Server durchzuführen. Das Beatmungsgerät kann dabei in Abhängigkeit der Bewertung der Systemzeit eine Anmeldung abzulehnen. Der Server ist eingerichtet die Bewertung an das Beatmungsgerät zu senden. Basierend auf der gesendeten Bewertung des Servers ist das Beatmungsgerät eingerichtet, eine Anmeldung des Beatmungsgerätes zur Datenübertragung an dem Server durchzuführen. Ergibt die Bewertung des Servers, dass die Systemzeit abweicht oder außerhalb des Toleranzfensters liegt, ist das Beatmungsgerät eingerichtet, eine Anmeldung am Server zur Datenübertragung abzulehnen. Befindet sich die Systemzeit des Beatmungsgerätes innerhalb des Toleranzfensters bzw. weicht diese nicht von der Server-Zeit ab, ist das Beatmungsgerät eingerichtet, eine Anmeldung am Server zur Daten Übertragung zuzulassen.

In einer Weiterbildung wird die Systemzeit durch einen Nutzer manuell neu festgelegt, wobei der Server das Beatmungsgerät ansteuert, dem Nutzer einen Hinweis zur Festlegung der Systemzeit zu geben, sodass eine definierte Übereinstimmung mit der Server-Zeit erreicht ist. Beispielsweise erfolgt ein Hinweis zur Festlegung der Systemzeit an den Nutzer, wenn die Systemzeit sich außerhalb des Toleranzfensters befindet oder von der Server-Zeit abweicht.

In einer weiteren Weiterbildung ist das Beatmungsgerät eingerichtet, basierend auf der Bewertung der Systemzeit durch den Server von einem Anwender eine Systemzeitkorrektur zu fordern, um die Anmeldung am Server durchzuführen. Erst nach manueller Festlegung der neuen Systemzeit durch den Nutzer und erneuter Bewertung der Systemzeit durch den Server und einer Übereinstimmung der Systemzeit mit der Server-Zeit kann eine Anmeldung des Beatmungsgerätes am Server erfolgen.

In einer weiteren Ausgestaltung ist das System eingerichtet, eine Einstellung der Systemzeit durch den Nutzer zu sperren, wenn das Beatmungsgerät einmal erfolgreich angemeldet ist. Dies bietet den Vorteil, dass die Systemzeit durch einen Nutzer nicht ungewollt/gewollt verstellbar ist. In der Regel ist die Systemzeit jedoch auf einem Prüfstand oder über einen Servicezugang am Gerät einstellbar. In der Regel kann die Systemzeit nicht vor das letzte gespeicherte Therapieende zurückgestellt werden.

In einer weiteren Ausgestaltung ist das Beatmungsgerät eingerichtet, die Daten und die Systemzeit über die Schnittstelle an den Server zu versenden. Das Beatmungsgerät ist in der Regel eingerichtet eine Schnittstelle zu umfassen, die zur Datenübertragung geeignet ist.

In einer weiteren Ausgestaltung werden bei einer definierten Übereinstimmung der Server-Zeit mit der Systemzeit die Daten und die Systemzeit von dem Beatmungsgerät übertragen und in einem Serverspeicher ablegt. Durch die Übertragung der Daten und der Systemzeit von dem Beatmungsgerät kann der Speicherplatz, den diese Daten auf dem Beatmungsgerät eingenommen haben, wieder freigegeben werden.

In einer Weiterbildung erhält das Beatmungsgerät zumindest zeitweise einen Zeitstempel von einem Zeit-Server. In der Regel ist das Beatmungsgerät eingerichtet zumindest einmal täglich einen Zeitabgleich seiner Systemzeit mit einer Server-Zeit eines Zeitservers durchzuführen. Alternativ können andere Abgleichintervalle einstellbar sein. Beispielsweise kann eine Abgleichung wöchentlich erfolgen.

In einer weiteren Weiterbildung stellt der Zeit-Server über das Internet oder Mobilfunknetz oder lokalen Funknetz (WIFI, Bluetooth) den Zeitstempel bereit oder ist ein PC oder eine Funkuhr oder ein Gerät. Dies bietet den Vorteil, dass je nach lokalen Gegebenheiten ein bestmöglicher Zugang des Beatmungsgerätes zu einem Server ermöglicht wird.

In einer Weiterbildung weist das Beatmungsgerät alternativ oder ergänzend zu der Zeiteinheit einen internen Zähler auf, der fortlaufend neue Zählerstände erzeugt, die gemeinsam mit den Daten gespeichert werden, wobei bei jeder Datenübertragung zu dem Server der aktuelle Zählerstand übermittelt wird. Dadurch kann bei jeder Datenübertragung durch Übermittlung des aktuellen Zählerstandes der Server, der die aktuelle Server-Zeit kennt, eine Zuordnung zwischen Zählerstand und Datum ermitteln.

In einer Weiterbildung gleicht der Server den aktuellen Zählerstand mit der Server-Zeit ab und nimmt eine Zuordnung von Zählerstand zu Server-Zeit vor und ordnet die Daten, und damit die den Daten zugeordneten, alten Zählerstände, ebenfalls entsprechend der Server-Zeit chronologisch zu. Dadurch ist es möglich, den internen Zählerstand mit der Server-Zeit abzugleichen.

Gegenstand der vorliegenden Erfindung ist zudem ein System zur Datenübertragung zwischen einem Server und einem Beatmungsgerät, wobei das Beatmungsgerät aufweist; eine Quelle für Atemgas und zumindest eine Schnittstelle zur Ermöglichung einer Datenübertragung, eine Vorrichtung zur Ermittlung von Daten, eine Steuereinheit, einen Speicher und eine Zeiteinheit zur Festlegung einer Systemzeit.

Erfindungsgemäß legt das Beatmungsgerät die Daten zumindest zeitweise in dem Speicher ab und ordnet den Daten die Systemzeit als Zeitstempel zu, wobei die Systemzeit über die Schnittstelle an den Server versendet wird, wobei der Server eine Server-Zeiteinheit zur Bereitstellung einer Server-Zeit und eine Vergleichseinrichtung aufweist, wobei die Vergleichseinrichtung die Server-Zeit mit der Systemzeit vergleicht und bei einer definierten Übereinstimmung der Server-Zeit mit der Systemzeit die Daten von dem Beatmungsgerät überträgt und in dem Serverspeicher ablegt und wobei der Server eingerichtet ist, eine Abweichung zwischen der Systemzeit und der Server-Zeit zu bewerten und eine entsprechende Bewertung an das Beatmungsgerät zu übermitteln, wobei das Beatmungsgerät bei einer definierten Abweichung der Server-Zeit von der Systemzeit eine Übertragung von Daten von dem Beatmungsgerät sperrt, bis die Systemzeit derart neu festgelegt wird, dass eine definierte Übereinstimmung mit der Server-Zeit erreicht ist, wobei die neu festgelegte Systemzeit derart auf den Zeitstempel in dem Speicher des Beatmungsgerätes angewandt wird, dass den Daten ein neuer Zeitstempel zugeordnet wird, woraufhin die Daten mit dem neuen Zeitstempel an den Server übertragen werden. Dies bietet den Vorteil, dass Daten, beispielsweise Patientendaten, bei einer Telemonitoring/einer Datenübertragung mit einem korrekten Zeitstempel versehen sind und dadurch bei der weiteren Verarbeitung korrekt zugeordnet werden können. In der Regel erhalten bereits gespeicherte Daten ihren Zeitstempel und zukünftig erzeugte Daten werden mit einem neuen Zeitstempel gemäß dem neuen Datum versehen. Optional kann das System ein Toleranzfenster vorgeben, bei dem eine abweichende Systemzeit als korrekt bewertet wird. Ein Toleranzfenster kann beispielsweise zwischen 0,1 Sekunden und 1 Minute betragen. Bei einer vorliegenden Abweichung wird einem Nutzer typischerweise ein Hinweis angezeigt, die Systemzeit einzustellen, bevor mit einer Übertragung der Daten begonnen werden kann.

Ein weiterer Gegenstand ist ein System zur Datenübertragung zwischen einem Server und einem Beatmungsgerät, wobei das Beatmungsgerät aufweist; eine Quelle für Atemgas und zumindest eine Schnittstelle zur Ermöglichung einer Datenübertragung, eine Vorrichtung zur Ermittlung von Daten, eine Steuereinheit, einen Speicher, eine Zeiteinheit zur Festlegung einer Systemzeit.

Das Beatmungsgerät ist eingerichtet, die Daten mit einem Zeitstempel zu versehen, zu speichern und zu einem späteren Zeitpunkt zu übertragen, wobei der Zeitstempel nicht oder begrenzt auf ein Toleranzfenster einstellbar eingerichtet ist, wobei das System eingerichtet ist, basierend auf dem Zeitstempel einen Abgleich einer Systemzeit des Beatmungsgerätes mit einer Server-Zeit des Servers durchzuführen, wenn die Datenübertragung gestartet wird, wobei die Systemzeit manuell einstellbar ist, bevor das Beatmungsgerät am Server angemeldet wird, wobei der Server eingerichtet ist, die manuell eingestellte Systemzeit vor einer Anmeldung am Server zu bewerten und mit der Server-Zeit zu vergleichen. Der Server ist eingerichtet, bei der ersten Anmeldung die Systemzeit durch Vergleich mit der Serverzeit zu kontrollieren. Weicht die Systemzeit vom Toleranzfenster ab, so wird der Anwender gezwungen, vor der Anmeldung am Server die Zeit zu korrigieren.

Ein weiterer Gegenstand ist ein System zur Datenübertragung zwischen einem Server und einem Beatmungsgerät, wobei das Beatmungsgerät aufweist; eine Quelle für Atemgas und zumindest eine Schnittstelle zur Ermöglichung einer Datenübertragung, eine Vorrichtung zur Ermittlung von Daten, eine Steuereinheit, einen Speicher, eine Zeiteinheit zur Festlegung einer Systemzeit.

Das Beatmungssystem ist eingerichtet, die Daten mit einem Zeitstempel zu versehen, zu speichern und zu einem späteren Zeitpunkt zu übertragen, wobei der Zeitstempel nicht oder begrenzt auf ein Toleranzfenster einstellbar eingerichtet ist, wobei das System eingerichtet ist, basierend auf dem Zeitstempel einen Abgleich einer Systemzeit des Beatmungsgerätes mit einer Server-Zeit des Servers durchzuführen, wenn die Datenübertragung gestartet wird, wobei das Beatmungsgerät eine Anzeige-zeit umfasst, die eingerichtet ist, sich von der Systemzeit bis zu 24 Stunden zu unterscheiden. Damit wird dem Nutzer ermöglicht, an einem Display des Beatmungsgerätes die korrekte lokale Uhrzeit (Zeitzone) angezeigt zu bekommen und zwischen Winter- und Sommerzeit zu wechseln. In der Regel wird die Systemzeit zu den gespeicherten Daten als Zeitstempel gespeichert. Optional ist die Anzeige-Zeit (oder ein Delta zwischen der Anzeige-Zeit und der Systemzeit) speicherbar, so dass beim Anschauen und Auswerten der Daten zusätzlich die Information über die lokale Uhrzeit des Anwenders / Patienten einsehbar ist.

Im Folgenden werden anhand von stark vereinfachten schematischen Darstellungen bevorzugte Ausführungsbeispiele näher erläutert. Es zeigt:
- Fig.1: einen grundsätzlichen Aufbau eines Beatmungsgeräts,
- Fig. 2: eine schematische Darstellung des erfindungsgemäßen Systems zur Datenübertragung.

In den Figuren weisen dieselben konstruktiven Elemente jeweils dieselben Bezugsziffern auf.

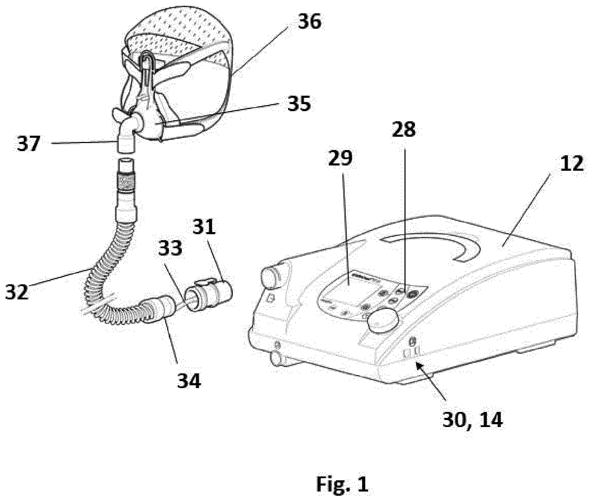

Figur 1 zeigt den grundsätzlichen Aufbau eines Beatmungsgerätes 12. Im Bereich eines Gerätegehäuses mit Bedienfeld 28 sowie Anzeige 29, ist in einem Geräteinnenraum eine Atemgaspumpe angeordnet. Über eine Kopplung 31 wird ein Verbindungsschlauch 32 angeschlossen. Entlang des Verbindungsschlauches 32 kann ein zusätzlicher Druckmessschlauch 33 verlaufen, der über einen Druckeingangsstutzen 34 mit dem

Gerätegehäuse verbindbar ist. Zur Ermöglichung einer Datenübertragung weist das Gerätegehäuse 12 zumindest eine Schnittstelle 30,14 auf. Ein Anfeuchter kann adaptiert werden.

Im Bereich einer dem Gerätegehäuse 12 abgewandten Ausdehnung des Verbindungsschlauches 32 ist ein Ausatmungselement angeordnet. Ebenfalls kann ein Ausatemventil verwendet werden.

Figur 1 zeigt darüber hinaus ein als Beatmungsmaske 10 ausgebildetes Patienten-Interface 35, das hier beispielsweise als Nasalmaske realisiert ist. Eine Fixierung im Bereich eines Kopfes eines Patienten kann über eine Kopfhaube 36 erfolgen. Im Bereich seiner dem Verbindungsschlauch 32 zugewandten Ausdehnung weist das Patienten-Interface 35 ein Kupplungselement 37 auf.

Über die Schnittstelle 30, 14 kann die Eingabe und / oder Ausgabe von Daten, wie beispielsweise Totraumvolumen, erfolgen. Die Schnittstellen 14, 30 können kabelgebunden, als Infrarot-Schnittstelle, als Bluetooth-Schnittstelle, Mobilfunk-Schnittstelle, IoT bzw. M2M Schnittstelle oder USB realisiert sein.

Das Beatmungsgerät 12 ist so ausgelegt, dass es über einen Schlauch und ein Patienteninterface 10 mit einem Patienten verbunden werden kann, um eine Beatmung bereitzustellen. Es umfasst eine Quelle für Atemgas, die beispielsweise als ein Elektromotor mit Gebläserad ausgebildet ist und eine Einrichtung zur Ermittlung von Druck und/oder Fluss und/oder Volumen des Atemgases, sowie eine Steuereinheit, die so ausgelegt ist, dass sie für jeden Atemzyklus auf der Basis eines vorbestimmten Wertes für den Patienten und/oder auf Basis von Messsignalen für die Parameter Druck und/oder Flow und/oder Volumen einen Atemgasdruck bestimmt und die Quelle für Atemgas derart reguliert, dass der Atemgasdruck erzeugt wird.

Das Beatmungsgerät 10 hat einen Speicher zur Speicherung von Daten, die die Nutzungsdauer und die Therapiequalität und ergänzend die Funktion / Wartung des Gerätes repräsentieren.

Die Steuereinheit ist ferner so ausgelegt, dass sie den aktuellen Druck und/oder Flow und/oder das Volumen von Atemgas bestimmt und den aktuellen Wert über eine mit der Steuereinheit verbundene Anzeige darstellt. Die Steuereinheit ist außerdem so ausgelegt, dass sie bezogen auf einen oder mehrere Parameter Trendveränderungen ihrer Berechnungen über der Zeit bestimmt, wobei die Trendveränderungen auf der Anzeige angezeigt werden können.

Über ein Modem oder eine andere Schnittstelle 30, 14 können ebenfalls aufgezeichnete Daten übermittelt werden.

Das in Figur 1 gezeigte Beatmungsgerät 12 ist zur Verwendung in einem erfindungsgemäßen System 10 zur Datenübertragung geeignet.

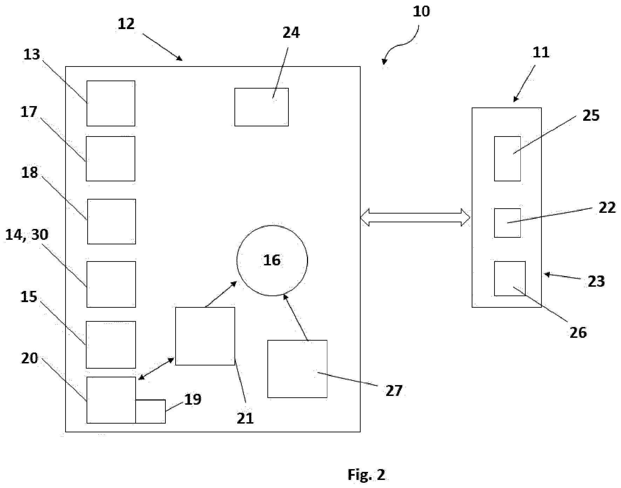

Figur 2 zeigt eine schematische Darstellung des erfindungsgemäßen Systems 10 zur Datenübertragung. Das erfindungsgemäße System umfasst den Server 11 sowie das Beatmungsgerät 12. Das Beatmungsgerät 12 umfasst mindestens eine Quelle für Atemgas 13, mindestens eine Schnittstelle 14, 30 zur Ermöglichung einer Datenübertragung, eine Vorrichtung 15 zur Ermittlung von Daten 16, eine Steuereinheit 17, einen Speicher 18 sowie eine Zeiteinheit 19 zur Festlegung einer Systemzeit 20. Das Beatmungsgerät 12 ordnet den Daten 16 die Systemzeit 20 als Zeitstempel zu und legt die erfassten Daten in einem Speicher 18 ab. Die Systemzeit 20 oder die Daten 16 mit dem Zeitstempel 21 werden in der Regel über die Schnittstelle 20 an den Server 11 übermittelt.

Der Server 11 umfasst eine Server-Zeiteinheit 25 zur Bereitstellung einer Server-Zeit 22 und eine Vergleichseinrichtung 26. Die Vergleichseinrichtung 26 vergleicht die Server-Zeit 22 mit der Systemzeit 20 und überträgt bei einer definierten Übereinstimmung der Server-Zeit 22 mit der Systemzeit 20 die Daten 16 von dem Beatmungsgerät 12 und legt sie in dem Serverspeicher ab.

Bei einer auftretenden Abweichung der Server-Zeit 22 von der Systemzeit 20 sperrt das Beatmungsgerät 10 eine Übertragung von Daten 16 von dem Beatmungsgerät 10 bis die Systemzeit 20 neu festgelegt wird, die eine definierte Übereinstimmung mit der Server-Zeit 22 erreicht. Das Beatmungsgerät 10 kann ein Toleranzfenster einrichten, in dessen Bereich eine Abweichung der Systemzeit 20 von der Server-Zeit 22 von dem Beatmungsgerät 10 toleriert wird. Bei einer vorliegenden Abweichung der Systemzeit 20 von der Server-Zeit 22 wird durch das Beatmungsgerät 10 ein Hinweis an einen Nutzer 38 des Beatmungsgerätes 10 ausgegeben, die Systemzeit 20 einzustellen.

Die neu festgelegte Systemzeit 20 wird auf den Zeitstempel 21 in dem Speicher 18 des Beatmungsgerätes 10 angewandt. Dabei wird den Daten 16 jeweils ein neuer Zeitstempel 21 zugeordnet. Die Daten 16 mit den neu zugeordneten Zeitstempeln 21 werden an den Server 11 übermittelt und erneut bewertet. Bei einer Übereinstimmung der neuen Systemzeit 20 mit der Server-Zeit 22 erfolgt eine Anmeldung des Beatmungsgerätes 10 an dem Server 11 sowie eine Übertragung der Daten 16 an den Server 11.

### Bezugszeichenliste

- 10: System
- 11: Server
- 12: Beatmungsgerät
- 13: Quelle für Atemgas
- 14, 30: Schnittstelle
- 15: Vorrichtung zur Ermittlung von Daten
- 16: Daten
- 17: Steuereinheit
- 18: Speicher
- 19: Zeiteinheit
- 20: Systemzeit
- 21: Zeitstempel
- 22: Server-Zeit
- 23: Zeit-Server
- 24: interner Zähler
- 25: Server-Zeiteinheit
- 26: Vergleichseinrichtung
- 27: neuer Zeitstempel
- 28: Bedienvorrichtung
- 29: Anzeige
- 30,14: Schnittstelle
- 31: Kopplung
- 32: Verbindungsschlauch
- 33: Druckmessschlauch
- 34: Druckeingangsstutzen
- 35: Patienten-Interface
- 36: Kopfhaube
- 37: Kupplungselement
- 38: Nutzer

## Patentansprüche

1. System (10) zur Datenübertragung zwischen einem Server (11) und einem Beatmungsgerät (12), wobei das Beatmungsgerät (12) aufweist; eine Quelle für Atemgas (13) und zumindest eine Schnittstelle (14) zur Ermöglichung einer Datenübertragung, eine Vorrichtung (15) zur Ermittlung von Daten (16), eine Steuereinheit (17), einen Speicher (18), eine Zeiteinheit (19) zur Festlegung einer Systemzeit (20), **dadurch gekennzeichnet, dass** das Beatmungsgerät (12) die Daten (16) zumindest zeitweise in dem Speicher (18) ablegt und den Daten die Systemzeit (20) als Zeitstempel (21) zuordnet, wobei die Systemzeit (20) über die Schnittstelle (14) an den Server (11) versendet wird, wobei der Server (11) eine Server-Zeiteinheit (25) zur Bereitstellung einer Server-Zeit (22) und eine Vergleichseinrichtung (26) aufweist, wobei die Vergleichseinrichtung (26) die Server-Zeit (22) mit der Systemzeit (20) vergleicht und bei einer definierten Übereinstimmung der Server-Zeit (22) mit der Systemzeit (20) die Daten (16) von dem Beatmungsgerät (12) überträgt und in einem Serverspeicher ablegt und wobei der Server (11) eingerichtet ist, eine Abweichung zwischen der Systemzeit (20) und der Server-Zeit (22) zu bewerten und eine entsprechende Bewertung an das Beatmungsgerät (12) zu übermitteln,
wobei das Beatmungsgerät (12) bei einer definierten Abweichung der Server-Zeit (22) von der Systemzeit (20) eine Übertragung von Daten (16) von dem Beatmungsgerät (12) sperrt, bis die Systemzeit (20) derart neu festgelegt wird, dass eine definierte Übereinstimmung mit der Server-Zeit (22) erreicht ist, wobei die neu festgelegte Systemzeit (20) derart auf den Zeitstempel (21) in dem Speicher (18) des Beatmungsgerätes (12) angewandt wird, dass den Daten (16) ein neuer Zeitstempel (27) zugeordnet wird, woraufhin die Daten (16) mit dem neuen Zeitstempel (27) an den Server (11) übertragen werden.

## Claims

1. A system (10) for transferring data between a server (11) and a ventilator (12), wherein the ventilator (12) comprises: a source for breathing gas (13) and at least one interface (14) for allowing for data transfer, a device (15) for gathering data (16), a control unit (17), a memory (18), a time unit (19) for setting a system time (20), **characterized in that** the ventilator (12) stores the data (16) in the memory (18) at least temporarily and assigns the system time (20) to the data as a time stamp (21), wherein the system time (20) is sent via the interface (14) to the server (11), wherein the server (11) comprises a server time unit (25) for providing a server time (22) and a comparison apparatus (26), wherein the comparison apparatus (26) compares the server time (22) with the system time (20) and, in the event of a defined match between the server time (22) and the system time (20), transfers the data (16) from the ventilator (12) and stores said data in a server memory and wherein the server (11) is configured to evaluate a deviation between the system time (20) and the server time (22) and to transfer a corresponding evaluation to the ventilator (12),
wherein the ventilator (12) blocks transfer of data (16) from the ventilator (12) in the event of a defined deviation between the server time (22) and the system time (20) until the system time (20) is newly set such that a defined match with the server time (22) is achieved, wherein the newly set system time (20) is applied to the time stamp (21) in the memory (18) of the ventilator (12) such that a new time stamp (27) is assigned to the data (16), whereupon the data (16) are transferred with the new time stamp (27) to the server (11).

## Revendications

1. Système (10) de transmission de données entre un serveur (11) et un appareil respiratoire (12), l'appareil respiratoire (12) comportant: une source de gaz respiratoire (13) et au moins une interface (14) pour permettre une transmission de données, un dispositif (15) pour la détermination de données (16), une unité de commande (17), une mémoire (18), une unité de temps (19) pour la définition d'un temps de système (20), **caractérisé en ce que** l'appareil respiratoire (12) enregistre les données (16) au moins temporairement dans la mémoire (18) et attribue aux données le temps de système (20) comme horodatage (21), le temps de système (20) étant envoyé au serveur (11) par l'interface (14), le serveur (11) comportant une unité de temps de serveur (25) pour la mise à disposition d'un temps de serveur (22) et un dispositif de comparaison (26), le dispositif de comparaison (26) comparant le temps de serveur (22) avec le temps de système (20) et, en cas de correspondance définie entre le temps de serveur (22) et le temps de système (20), transmettant les données (16) de l'appareil respiratoire (12) et les enregistrant dans une mémoire de serveur, et le serveur (11) étant configuré pour évaluer un écart entre le temps de système (20) et le temps de serveur (22) et pour transmettre une évaluation correspondante à l'appareil respiratoire (12),
l'appareil respiratoire (12) bloquant une transmission de données (16) de l'appareil respiratoire (12) en cas d'écart défini entre le temps de serveur (22) et le temps de système (20) jusqu'à ce que le temps de système (20) soit de nouveau défini de telle sorte qu'une correspondance définie avec le temps de serveur (22) est atteinte, le temps de système (20) de nouveau défini étant appliqué à l'horodatage (21) dans la mémoire (18) de l'appareil respiratoire (12) de telle sorte qu'un nouvel horodatage (27) est attribué aux données (16), à la suite de quoi les données (16) sont transmises au serveur (11) avec le nouvel horodatage (27).
